# EUROPÄISCHE PATENTSCHRIFT

(11) **EP 3 662 936 B1**
(45) Veröffentlichungstag und Bekanntmachung des Hinweises auf die Patenterteilung: **06.12.2023**
(21) Anmeldenummer: 18210836.5
(22) Anmeldetag: 06.12.2018
(51) Int. Cl.: A61L 2/22, A61C 19/00

(54) **REINIGUNGS- UND DESINFEKTIONSGERÄT MIT EINER FILTEREINRICHTUNG**
CLEANING AND DISINFECTING DEVICE WITH A FILTER DEVICE
APPAREIL DE NETTOYAGE ET DE DÉSINFECTION DOTÉ D'UN DISPOSITIF FILTRANT

(43) Veröffentlichungstag der Anmeldung: 10.06.2020
(73) Patentinhaber: MELAG Medizintechnik GmbH & Co. KG, 10829 Berlin (DE)
(72) Erfinder: Hering, Alexander, 12459 Berlin (DE); Litzba, Guido, 14513 Teltow (DE); Staerk, Michael, 12247 Berlin (DE)
(74) Vertreter: Maikowski & Ninnemann Patentanwälte Partnerschaft mbB

(56) Entgegenhaltungen:
- EP-A1- 1 433 412
- WO-A1-2015/028271
- DE-A1- 10 243 122
- DE-A1-102010 028 619
- US-A1- 2014 109 938

## Beschreibung

Die vorliegende Erfindung betrifft ein Reinigungs- und Desinfektionsgerät gemäß dem Oberbegriff des Anspruchs 1 sowie ein Verfahren zum Betrieb eines solchen Reinigungs- und Desinfektionsgeräts gemäß dem Oberbegriff des Anspruchs 11.

Einige medizinische Instrumente, wie etwa englumige Kanülen oder dentale Übertragungsinstrumente stellen besondere Anforderungen an die Wasserqualität der Reinigungsflotte eines Reinigungs-/Desinfektionsgerätes. Insbesondere im dentalen Anwendungsbereich befinden sich häufig Reste von Zwei-Komponenten-Zahnzement, Abdruckmasse zur Gebissabformung oder Desinfektionsmittel für Wurzelkanalbehandlungen an den aufzubereitenden Instrumenten. Folglich besteht das Problem, dass sich in der den aufzubereitenden Instrumenten zugeführten Spül- und Reinigungsflüssigkeit Rückstände befinden, die sich in den fein strukturierten Instrumenten bzw. engen Durchgangskanälen und Getriebehohlräumen dieser Instrumente festsetzen und aushärten können. Dies führt mitunter zu einer kostspieligen Beschädigung der teuren medizinischen und zahnmedizinischen Instrumente.

Gleichzeitig besteht der Wunsch neben einer guten Mikrofilterung der Waschflotte möglichst lange Filterstandzeiten zu erzielen, um Material- bzw. Personalkosten, die mit dem Unterhalt von Filtereinrichtungen verbunden sind, gering zu halten.

Zusätzlich zu den Anforderungen an die Wasserqualität der Waschflotte gibt es die Erwartungen an eine möglichst gute Innentrocknung der aufzubereitenden Instrumente. Befindet sich im Strömungsweg zu einem Instrument ein Filterelement, so muss die Trocknungsluft, die für die Innentrocknung eingeleitet wird, den Strömungswiderstand des Filterelementes überwinden, um zu dem Instrument zu gelangen.

Mikrofilter sind bei Reinigungs- und Desinfektionsgeräten meist dezentral unmittelbar vor Instrumentenadaptern oder zentral in Verteilerleisten in der Beladung integriert. Die Filter unterliegen keiner Überwachung durch die Gerätesteuerung, sondern müssen nach festen Zeitintervallen durch den Anwender überprüft und ggf. erneuert oder gereinigt werden. Je nach Intensität des Schmutzeintrages müssen diese Wechselintervalle sehr kurz gewählt werden, damit es nicht zu einem vollständigen Zusetzen des Filters kommt und so die Reinigungs- und Desinfektionswirkung beeinträchtigt wird.

Bei diesen Anordnungen befindet sich der Mikrofilter im Strömungsweg zu einem Instrument, so dass neben der Waschflotte bei der Reinigung und Desinfektion auch die Trocknungsluft in der Trocknungsphase des Gerätes den Strömungswiderstand des Filterelementes überwinden muss, um zu dem Instrument zu gelangen.

Aus der DE 10 2010 028 619 A1 ist ein Ventil für ein Haushaltsgerät bekannt, das ein beweglich gelagertes Filterelement aufweist. Durch eine Bewegung des Filterelements kann dieses von seiner ersten Seite oder von seiner zweiten Seite aus durchspült werden. Dadurch wird eine Reinigung des Filterelements mittels Gegenspülen ermöglicht. Dabei ist das Filterelement derart angeordnet, dass es stets von einer Flüssigkeit durchströmt werden muss, die durch das Ventil durchgeführt wird.

Aus der DE 10 2007 061 038 A1 ist eine Geschirrspül- oder Waschmaschine mit einem zwangsdurchströmten selbstreinigenden Filtersystem bekannt. Dieses Filtersystem weist einen Mikrofilter auf, um auch kleine Partikel zurückzuhalten und so eine Wiederanschmutzung von Reinigungsgut zu verhindern.

Aus der DE 10 2011 087 202 B3 ist ein Haushaltsgerät mit einer Filtereinrichtung bekannt, bei der ein Filterelement verschwenkt werden kann, um es wahlweise von der einen oder von der anderen Seite aus durchzuspülen.

Aus der EP 2 896 342 A1 ist eine Verteilerleiste eines Spülgerätes bekannt, die einen Zentralfilter für sämtliche Anschlussstellen der Verteilerleiste aufweist.

Aus der WO 2011/076685 A1 ist eine Filtervorrichtung zum Filtern einer Flüssigkeit eines Haushaltsgerätes bekannt. Dabei kann eine Flüssigkeit, die die Filtervorrichtung durchströmt, wahlweise gefiltert oder nicht gefiltert werden. Ferner ist ein Ventil in einer Leitung vorgesehen, durch die die filtrierte Flüssigkeit strömt.

Aus der DE 10 2012 103 435 A1 ist ein Geschirrspüler mit einem sich drehenden Filter bekannt. Dabei ist ein Abstreifelement vorgesehen, das Verunreinigungen von dem sich drehenden Filter abstreift.

Aus der EP 1 433 412 A1 ist eine Vorrichtung zum Reinigen von Endoskopen bekannt. Dabei kann eine durch einen Filter gereinigte Reinigungsflüssigkeit einer Außenseite eines zu reinigenden Endoskops und zusätzlich optional einem Innenlumen des Endoskops zugeführt werden. Die Vorrichtung weist Reinigungsarme zum äußeren Besprühen von medizinischen Instrumenten sowie Anschlüsse und Leitungen zum Durchspülen der Instrumentenkanäle auf, wobei die Leitungen zum Durchspülen der Instrumentenkanäle mit einem Ventil ausgestattet sind. Den Leitungen zur Innen- und Außenreinigung sind jeweils unterschiedliche Filter, wie Grob- und Feinfilter, zum Reinigen des umgepumpten Spülwassers zugeordnet.

Somit sind aus dem Stand der Technik zwar Haushaltsgeräte bekannt, bei denen Einrichtungen vorgesehen sind, um verunreinigte Filter wieder zu reinigen bzw. um eine Spülflüssigkeitsströmung durch ein Filterelement zu beeinflussen. Allerdings weisen die aus dem Stand der Technik bekannten Lösungen einige Nachteile auf.

Beispielsweise wird dann, wenn ein Filter im Strömungsweg eingesetzt wird, regelmäßig nur eine schlechte Innentrocknung von aufzubereitenden Instrumenten erreicht, da ein verhältnismäßig großer Strömungswiderstand der verwendeten Filter auch von der Trocknungsluft überwunden werden muss.

Darüber hinaus weisen einige der aus dem Stand der Technik bekannten Lösungen ein schlechtes Verhältnis zwischen der Filterstandzeit (also der Lebensdauer eines Filters) und dem für den Filter benötigten Bauraum auf. Dies ist insbesondere dadurch begründet, dass entweder keine selbstreinigende Funktion der Filtereinrichtungen vorgesehen ist oder aber die bei einer Selbstreinigung anfallende Flüssigkeit nicht sinnvoll weiter verwendet werden kann.

Schließlich weisen einige aus dem Stand der Technik bekannte Lösungen einen hohen Betreuungsaufwand auf. Zudem kann eine mangelnde Achtsamkeit des Personals, das die mit den Filtereinrichtungen versehenen Geräte bedient, schnell zu einer Fehlfunktion führen. Dies betrifft insbesondere mitunter schwierig durchzuführende Schritte einer Filterreinigung.

Der vorliegenden Erfindung liegt die Aufgabe zugrunde, ein Reinigungs- und Desinfektionsgerät anzugeben, das die aus dem Stand der Technik bekannten Nachteile überwindet und insbesondere eine benutzerfreundliche, zuverlässige und qualitativ gleichbleibende Filtration von Spülflüssigkeit bei gleichzeitig guter Innentrocknung aufzubereitender Medizinprodukte oder Instrumente ermöglicht. Die Erfindung wird durch die Ansprüche 1 und 11 definiert.

Diese Aufgabe wird durch ein Reinigungs- und Desinfektionsgerät mit den Merkmalen des Anspruchs 1 gelöst. Ein solches Reinigungs- und Desinfektionsgerät dient der Reinigung und Desinfektion medizinischer und/oder zahnmedizinischer Instrumente. Es weist eine Filtereinrichtung zum Filtrieren von Spülflüssigkeit auf. Diese Filtereinrichtung weist ein Filterelement mit einer Rohseite und einer Reinseite auf. Durch einen Einlass der Filtereinrichtung kann wahlweise Spülflüssigkeit oder Trocknungsluft in die Filtereinrichtung eingeführt werden. Durch einen ersten Auslass und einen zweiten Auslass kann die Spülflüssigkeit bzw. die Trocknungsluft die Filtereinrichtung wieder verlassen. Dabei stehen der Einlass und der zweite Auslass mit der Rohseite des Filterelements in Strömungsverbindung. Das heißt, Spülflüssigkeit, die durch den Einlass in die Filtereinrichtung eingeführt wird, kann diese unfiltriert durch den zweiten Auslass wieder verlassen. Der erste Auslass steht mit der Reinseite des Filterelements in Strömungsverbindung. Spülflüssigkeit, die durch den Einlass in die Filtereinrichtung eingeführt wird, kann die Filtereinrichtung durch den ersten Auslass nur in filtrierter Form verlassen.

Erfindungsgemäß ist vorgesehen, dass der erste Auslass mit einer Vorrichtung zum Durchspülen bzw. Reinigen von Innenlumina medizinischer und/oder zahnmedizinischer Instrumente in Strömungsverbindung steht. Da die Innenlumina derartiger Instrumente leicht verstopfen können, wenn sich Partikel in der Spülflüssigkeit befinden, ist es sinnvoll, die Innenlumina nur mit filtrierter Spülflüssigkeit zu spülen. Demgegenüber kann der zweite Auslass mit einer Vorrichtung zum Reinigen von Außenseiten medizinischer und/oder zahnmedizinischer Instrumente in Strömungsverbindung gebracht werden. Um den zweiten Auslass mit einer solchen Vorrichtung zum Reinigen von Außenseiten in Verbindung zu bringen oder aber eine entsprechende Strömungsverbindung zu unterbrechen, ist am zweiten Auslass bzw. in einem durch den zweiten Auslass führenden Strömungspfad zudem eine Ventileinrichtung angeordnet. Ist die Ventileinrichtung geöffnet, gestattet sie ein Strömen von Spülflüssigkeit von dem Einlass der Ventileinrichtung durch den zweiten Auslass hindurch. Ist die Ventileinrichtung hingegen geschlossen, verhindert sie ein Strömen von Spülflüssigkeit durch den zweiten Auslass.

Diese Ausgestaltung ermöglicht eine Standzeitverlängerung des Filterelements, da die Spülflüssigkeit, die zur Vorrichtung zum Reinigen von Außenseiten medizinischer und/oder zahnmedizinischer Instrumente hinströmt, Schmutzpartikel, die sich an der Rohseite des Filterelements abgelagert haben, abtransportiert. Ein Beaufschlagen der Außenseiten der zu reinigenden medizinischen und/oder zahnmedizinischen Instrumente mit solchen Schmutzpartikeln ist grundsätzlich unproblematisch. Allerdings muss vermieden werden, dass diese Schmutzpartikel in die Innenlumina derartiger Instrumente eindringen. Durch die vorgeschlagene Anordnung wird genau das vermieden. So kann nur filtrierte Spülflüssigkeit in die Innenlumina der zu reinigenden Instrumente gelangen, während Schmutzpartikel, die sich auf der Rohseite des Filterelements abgelagert haben, fortwährend durch die Spülflüssigkeit abtransportiert werden, wenn diese entlang der Rohseite des Filterelements zur Vorrichtung zum Reinigen der Außenseiten der aufzubereitenden Instrumente strömt. Somit wird keine Spülflüssigkeit "verschwendet", sondern stets zur Reinigung der aufzubereitenden Instrumente eingesetzt.

Dabei dient die Ventileinrichtung dazu, das Strömen der Spülflüssigkeit zur Außenseite der aufzubereitenden Instrumente zu steuern. Auf diese Weise kann ein erhöhter Strömungswiderstand, der durch das Filterelement aufgebracht wird, kompensiert werden. Gleichzeitig ist es durch die Ventileinrichtung möglich, eine effektive Trocknung der Innenlumina der aufzubereitenden Instrumente zu gewährleisten. Denn solange die Ventileinrichtung geschlossen ist, muss Trocknungsluft zwangsweise durch das Filterelement zu den Innenlumina der aufzubereitenden Instrumente strömen. Dadurch wird ein höherer Trocknungsdruck erreicht und eine bessere Trocknung der Innenlumina ermöglicht. Folglich wird in diesen Innenlumina - unabhängig vom Strömungswiderstand des eingesetzten Filterelements - eine gute Trocknungsleistung erzielt. Demgegenüber sorgt der Strömungswiderstand eines solchen Filterelements bei den aus dem Stand der Technik bekannten Vorrichtungen regelmäßig für einen nur unzureichenden Trocknungserfolg in Innenlumina aufzubereitender Instrumente, da zusätzliche Strömungspfade vorgesehen sind, durch die die Trocknungsluft strömen kann.

Die Spülflüssigkeit kann beispielsweise Wasser oder mit einem Zusatz wie etwa einem Reinigungsmittel versehenes Wasser sein.

In einer Variante bildet die Vorrichtung zum Reinigen von Außenseiten medizinischer und/oder zahnmedizinischen Instrumente zugleich die Ventileinrichtung oder zumindest einen Teil davon. Es ist in dieser Variante also keine separate Ventileinrichtung erforderlich. Vielmehr kann die Vorrichtung zum Reinigen von Außenseiten medizinischer und/oder zahnmedizinischen Instrumente derart ausgestaltet sein, dass sie eine Strömung von Spülflüssigkeit durch sie hindurch gestattet oder verhindert.

In einer Variante handelt es sich bei der Vorrichtung zum Reinigen von Außenseiten medizinischer und/oder zahnmedizinischer Instrumente um einen Spülarm. Derartige Spülarme sind dem Fachmann hinlänglich bekannt und werden regelmäßig zur Reinigung von Außenseiten aufzubereitender Instrumente eingesetzt. Demgegenüber werden Innenlumina aufzubereitender Instrumente regelmäßig mithilfe einer Adapteranordnung wie etwa einer Adapterschiene, auf die die zu reinigenden Instrumente aufgesteckt werden, gereinigt. Dann fließt die Spülflüssigkeit durch die entsprechende Adapterschiene und durchströmt einen Innenkanal bzw. ein anderes Innenlumen des aufzubereitenden Instruments.

In einer Variante weist die Filtereinrichtung ein Gehäuse mit einem Deckel mit einer integrierten Spülarmaufnahme auf. Die Spülarmaufnahme dient dabei zum beweglichen Befestigen des Spülarms. In einer weiteren Variante wird die Ventileinrichtung durch den Spülarm und die Spülarmaufnahme gebildet. Dies kann besonders einfach dadurch bewerkstelligt werden, dass die Spülarmaufnahme nicht nur eine Rotationsbewegung des Spülarms erlaubt, sondern auch eine axiale Bewegung entlang einer Rotationsachse des Spülarms. Wird der Spülarm beispielsweise entlang der Rotationsachse angehoben, kann eine Strömung von Spülflüssigkeit durch den Spülarm hindurch ermöglicht werden (das Ventil in der Spülarmaufnahme ist dann geöffnet). Dieses Anheben des Spülarms kann beispielsweise durch die Spülflüssigkeit selbst erfolgen. Wenn die Spülflüssigkeit also mit einem ausreichend hohen Druck in die Filtereinrichtung eingebracht wird, kann sie selbst dafür sorgen, dass der Spülarm angehoben und das in der Spülarmaufnahme ausgebildete Ventil geöffnet wird. Sinkt hingegen der Spülflüssigkeitsdruck, kann sich der Spülarm automatisch durch die Schwerkraft wieder absenken und das Ventil wieder verschließen. Diese Öffnung des Ventils mithilfe des Spülflüssigkeitsdrucks und dieses Schließen des Ventils mithilfe der Schwerkraft gestattet eine besonders einfache Steuerung der Ventileinrichtung, ohne dass hierfür weitere Sensoren oder Aktoren zur Betätigung der Ventileinrichtung erforderlich wären.

In einer Variante liegt der Druck, ab dem die Ventileinrichtung geöffnet wird, in einem Bereich von 20 bis 200 mbar, insbesondere 30 bis 195 mbar, insbesondere 40 bis 190 mbar, insbesondere 50 bis 180 mbar, insbesondere 60 bis 170 mbar, insbesondere 70 bis 160 mbar, insbesondere 80 bis 150 mbar, insbesondere 90 bis 140 mbar, insbesondere 100 bis 130 mbar, insbesondere 110 bis 120 mbar.

In einer Variante ist die Ventileinrichtung derart ausgestaltet, dass sie ein Rückfließen von Spülflüssigkeit aus der Vorrichtung zum Reinigen von Außenseiten medizinischer und/oder zahnmedizinischer Instrumente gestattet. Dies gilt insbesondere dann, wenn diese Vorrichtung als Spülarm ausgestaltet ist, um ein Entleeren des Spülarms zu ermöglichen. Der Rückfluss der Spülflüssigkeit erfolgt dann in die Filtereinrichtung. Dabei kann die rückfließende Spülflüssigkeit die Filtereinrichtung durch den Einlass wieder verlassen.

In einer Variante ist das Filterelement als Ringfilterelement ausgestaltet. Ein solches Ringfilterelement weist eine Innenseite und eine Außenseite auf. Dabei ist vorgesehen, dass die Innenseite des Ringfilterelements die Rohseite des Filterelements darstellt. Demgegenüber stellt die Außenseite des Ringfilterelements die Reinseite des Ringfilterelements dar. Spülflüssigkeit, die durch das Filterelement filtriert werden soll, strömt dann von der Innenseite des Ringfilterelements zu dessen Au ßenseite und von dort weiter zur Vorrichtung zum Durchspülen von Innenlumina medizinischer und/oder zahnmedizinischer Instrumente weiter.

In einer Variante befinden sich der Einlass auf einer ersten Stirnseite des Ringfilterelements und der zweite Auslass auf einer zweiten Stirnseite des Ringfilterelements. Dabei ist die zweite Stirnseite der ersten Stirnseite gegenüberliegend angeordnet. Spülflüssigkeit, die durch den ersten Einlass in das Ringfilterelement eintritt, kann dieses auf der Rohseite durchqueren und das Ringfilterelement an der zweiten Stirnseite durch den zweiten Auslass verlassen.

In einer Variante weist die Ventileinrichtung einen Öffnungsdruck auf, der größer als ein Strömungswiderstand ist, der von dem Filterelement und dem ersten Auslass (und insbesondere auch von weiteren Elementen, die in Strömungsrichtung nach dem ersten Auslass angeordnet sind, wie etwa die Vorrichtung zum Durchströmen von Innenlumina medizinischer und/oder zahnmedizinischer Instrumente) gemeinsam erzeugt wird. Wenn Trocknungsluft in die Filtereinrichtung einströmt, würde sie aufgrund der geschlossenen Ventileinrichtung nur durch das Filterelement und den ersten Auslass hindurch zur Vorrichtung strömen, die dem Reinigen und Trocknen von Innenlumina medizinischer und/oder zahnmedizinischer Instrumente dient. So lässt sich eine spezifische Innentrocknung der medizinischen und/oder zahnmedizinischen Instrumente erreichen. Wenn dann der Druck der Trocknungsluft erhöht wird, öffnet sich die Ventileinrichtung, sodass die Trocknungsluft anschließend auch durch den zweiten Auslass hindurch aus der Filtereinrichtung austritt. Neben einer Innentrocknung der aufzubereitenden Instrumente erfolgt dann auch eine Außentrocknung der aufzubereitenden Instrumente.

Demgegenüber könnte Spülflüssigkeit bereits initial auch durch die Ventileinrichtung strömen, da der vom Massenstrom der Spülflüssigkeit aufgebrachte Druck regelmäßig ausreichend groß ist, um die Ventileinrichtung zu öffnen, selbst wenn der Öffnungsdruck der Ventileinrichtung größer als der Strömungswiderstand des Filterelements und des ersten Auslasses ist.

Wie bereits oben erwähnt, ist die Ventileinrichtung in einer Variante als passive, selbständig schaltende Ventileinrichtung ausgestaltet. Dabei kann eine Öffnung der Ventileinrichtung beispielsweise über einen Spülflüssigkeitsdruck bewerkstelligt werden. Demgegenüber kann ein Schließen der Ventileinrichtung beispielsweise durch die Schwerkraft erfolgen. Es kann aber auch eine Feder oder ein anderes Vorspann- bzw. Rückstellelement vorgesehen sein, das die Ventileinrichtung in ihrer geschlossenen Stellung hält und gegen das ein entsprechender Öffnungsdruck aufgebracht werden muss. Auch dieser Öffnungsdruck kann vorzugsweise durch die Spülflüssigkeit selbst aufgebracht werden.

In einer Variante handelt es sich bei der Ventileinrichtung um eine aktiv schaltende Ventileinrichtung, die mittels eines Steuergeräts des Reinigungs- und Desinfektionsgeräts angesteuert und aktiv geöffnet und/oder geschlossen wird. Es ist auch möglich, ein aktives Öffnen und passives Schließen oder ein passives Schließen und ein aktives Öffnen der Ventileinrichtung vorzusehen.

In einer Variante weist die Filtereinrichtung einen zusätzlichen Auslass in ihrem Bodenbereich auf. Durch diesen zusätzlichen Auslass kann dann rücklaufende Spülflüssigkeit aus der Filtereinrichtung wieder ausströmen. Ein solcher zusätzlicher Auslass ist insbesondere dann sinnvoll, wenn sich das Filterelement der Filtereinrichtung bereits etwas zugesetzt hat und einen Rückfluss von Flüssigkeit erschwert. Dann sorgt der zusätzliche Auslass dafür, dass sich keine Flüssigkeit in der Filtereinrichtung ansammelt, sondern aus dieser wieder austreten kann. Rücklaufende Spülflüssigkeit tritt immer dann in Erscheinung, wenn eine Umwälzpumpe des Reinigungs- und Desinfektionsgeräts abgeschaltet wird, beispielsweise nach Beendigung einer Reinigungsphase des in dem Reinigungs- und Desinfektionsgerät durchgeführten Reinigungsprogramms.

Durch den optional vorgesehenen zusätzlichen Auslass wird eine zusätzliche Verbindung zwischen der Reinseite und der Rohseite des Filterelements hergestellt. Um einen unerwünschten Übertritt von unfiltrierter Spülflüssigkeit von der Rohseite auf die Reinseite durch den zusätzlichen Auslass zu vermeiden, ist in einer Variante ein zusätzliches Filterelement im bzw. am zusätzlichen Auslass angeordnet. Dabei muss Spülflüssigkeit, die durch den zusätzlichen Auslass hindurchströmt, dieses zusätzliche Filterelement passieren. Durch den Einsatz eines solchen zusätzlichen Filterelements wird die Rohseite des Filterelements weiterhin sicher von der Reinseite des Filterelements getrennt. Rückfließende Spülflüssigkeit, die durch den zusätzlichen Auslass und das zusätzliche Filterelement zum Einlass der Filtereinrichtung strömt, führt dann automatisch zu einem Gegenspülen des zusätzlichen Filterelements, sodass sich Partikel, die sich auf der Rohseite des zusätzlichen Filterelements abgelagert haben, wieder entfernt werden.

Da im Betrieb der Filtereinrichtung typischerweise das Volumen der rückfließenden Spülflüssigkeit größer ist als das Volumen der Spülflüssigkeit, die nicht durch das eigentliche Filterelement, sondern durch das zusätzliche Filterelement strömt, wird das zusätzliche Filterelement stets sauber gehalten. Denn einem kleinen Volumen von Spülflüssigkeit, aus der durch das zusätzliche Filterelement Schmutzpartikel entfernt werden, steht ein großes Volumen von rückfließender, bereits filtrierter Spülflüssigkeit gegenüber, die für ein Gegenspülen des zusätzlichen Filterelements und damit für ein Entfernen dort auf der Rohseite befindlichen Schmutzpartikel sorgt.

In einer Variante ist am zusätzlichen Auslass ein Rückschlagelement vorgesehen. Dieses Rückschlagelement dient dazu, einen Flüssigkeitsstrom von der Reinseite zum Einlass der Filtereinrichtung zu gestatten, einen Flüssigkeitsstrom vom Einlass zur Reinseite des Filterelements jedoch zu verhindern. Durch ein derartiges Rückschlagelement wird die Wahrscheinlichkeit, dass sich Schmutzpartikel an einem optionalen zusätzlichen Filterelement im zusätzlichen Auslass anlagern, nochmals verringert. Dennoch kann ein solches zusätzliches Filterelement auch im Fall des Vorhandenseins eines Rückschlagelementes vorgesehen sein, um für eine erhöhte Sicherheit zu sorgen, dass tatsächlich keine unfiltrierte Spülflüssigkeit von der Rohseite des Filterelements zur Reinseite des Filterelements durch den zusätzlichen Auslass gelangt. Das Rückschlagelement soll also grundsätzlich einen Flüssigkeitsstrom von der Rohseite zur Reinseite durch den zusätzlichen Auslass verhindern. Wenn jedoch trotzdem geringe Mengen an Spülflüssigkeit durch den zusätzlichen Auslass in dieser Richtung strömen, sorgt das zusätzliche Filterelement dafür, dass auch diese Spülflüssigkeit filtriert wird.

Die Filtereinrichtung ist in einer Variante über eine Reinigungskammer des Reinigungs- und Desinfektionsgeräts leicht zugänglich, um einen einfachen Austausch bzw. ein einfaches Warten zu ermöglichen. Dabei ist es in einer Variante vorgesehen, dass die Filtereinrichtung werkzeugfrei aus der Reinigungskammer entnommen bzw. dort wieder eingesetzt werden kann.

Ein Aspekt der Erfindung betrifft ein Verfahren zum Betrieb eines Reinigungs- und Desinfektionsgeräts für medizinische und/oder zahnmedizinische Instrumente gemäß Anspruch 11. Wie ausgeführt, weist ein solches Reinigungs- und Desinfektionsgerät eine Filtereinrichtung zum Filtrieren von Spülflüssigkeit auf, wobei die Filtereinrichtung ein Filterelement mit einer Rohseite und einer Reinseite aufweist. Ferner ist ein Einlass zum wahlweisen Zuführen von Spülflüssigkeit oder Trocknungsluft in die Filtereinrichtung vorgesehen. Außerdem weist die Filtereinrichtung einen ersten Auslass und einen zweiten Auslass auf. Der Einlass und der zweite Auslass stehen mit der Rohseite des Filterelements in Strömungsverbindung. Der erste Auslass steht hingegen mit der Reinseite des Filterelements in Strömungsverbindung.

Das Verfahren zeichnet sich erfindungsgemäß dadurch aus, dass Spülflüssigkeit, die durch den Einlass in die Filtereinrichtung einströmt, durch das Filterelement hindurch und durch den ersten Auslass aus der Filtereinrichtung herausströmt und zu einer Vorrichtung zum Reinigen von Innenlumina medizinischer und/oder zahnmedizinischer Instrumente hinströmt. Darüber hinaus wird eine Ventileinrichtung in Abhängigkeit von Verfahrensparametern geöffnet, sodass die Spülflüssigkeit nach dem Öffnen der Ventileinrichtung nicht nur durch den ersten Auslass ausströmt, sondern zusätzlich auch durch den zweiten Auslass aus der Filtereinrichtung herausströmen und zu einer Vorrichtung zum Reinigen von Außenseiten medizinischer und/oder zahnmedizinischer Instrumente strömen kann. Dabei ist der zweite Auslass zunächst von der Ventileinrichtung verschlossen und wird erst in Abhängigkeit von Verfahrensparametern geöffnet.

In einer Variante wird die Ventileinrichtung in Abhängigkeit des Drucks der Spülflüssigkeit oder der Trocknungsluft, der Temperatur der Spülflüssigkeit oder der Trocknungsluft und/oder der Zeit, beispielsweise der seit Beginn des Reinigungsverfahrens verstrichenen Zeit, geöffnet und/oder geschlossen. Dieses Öffnen und/oder Schließen kann elektromechanisch oder rein mechanisch erfolgen. Das Öffnen und/oder Schließen kann aktiv (beispielsweise durch das Senden entsprechender Steuerungsbefehle) oder passiv erfolgen.

In einer Variante wird die Ventileinrichtung dann geöffnet, wenn ein Flüssigkeitsdruck der Spülflüssigkeit einen Schwellenwert überschreitet.

In einer Variante schließt sich die Ventileinrichtung automatisch, wenn der Flüssigkeitsdruck unter den Schwellenwert sinkt. Dieses automatische Schließen kann beispielsweise mithilfe der Schwerkraft erfolgen.

In einer Variante wird das Verfahren derart durchgeführt, dass Trocknungsluft in die Filtereinrichtung nur mit einem solchen Druck eingebracht wird, der unterhalb des Schwellenwertes liegt. Dann bleibt die Ventileinrichtung dauerhaft geschlossen, wenn die Trocknungsluft die Filtereinrichtung durchströmt. Auf diese Weise kann ein besonders vorteilhaftes Trocknen von Innenlumina medizinischer und/oder zahnmedizinischer Instrumente erreicht werden, die im Zuge des beanspruchten Verfahrens aufbereitet werden.

Weitere Details von Aspekten der vorliegenden Erfindung werden anhand von Ausführungsbeispielen und Figuren näher erläutert. Es zeigen:
- Figur 1: eine schematische Darstellung eines Ausführungsbeispiels einer Filtereinrichtung eines Reinigungs- und Desinfektionsgeräts;
- Figur 2A: ein Ausführungsbeispiel einer Spülarmanordnung in geschlossenem Zustand;
- Figur 2B: die Spülarmanordnung der Figur 2A in einem geöffneten Zustand;
- Figur 3A: eine erste schematische Darstellung der Flüssigkeitsströmung durch ein Ausführungsbeispiel einer Filtereinrichtung eines erfindungsgemäßen Reinigungs- und Desinfektionsgeräts; und
- Figur 3B: eine zweite schematische Darstellung der Flüssigkeitsströmung durch die Filtereinrichtung der Figur 3A.

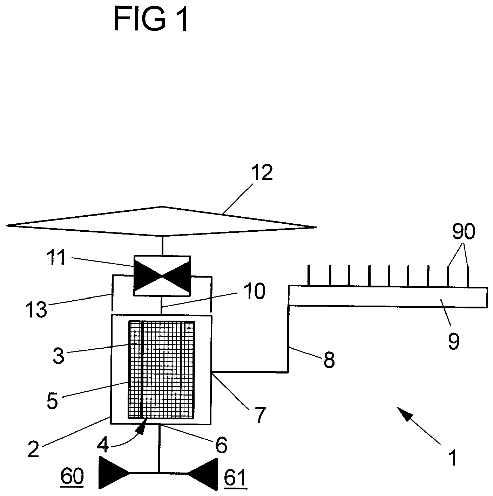

Die Figur 1 zeigt eine Filtereinrichtung 1 eines Reinigungs- und Desinfektionsgeräts. Die Filtereinrichtung 1 weist ein Gehäuse 2 und ein in dem Gehäuse 2 angeordneten Ringfilter 3 auf, der als Filterelement dient. Eine in der Figur 1 nicht sichtbare Innenseite 4 des Ringfilters 3 bildet die Rohseite des Filters 3. Eine Außenseite 5 des Ringfilters 3 bildet seine Reinseite.

Durch einen Einlass 6 kann wahlweise Spülflüssigkeit 60 oder Trocknungsluft 61 in das Gehäuse 2 geleitet werden, und zwar nur auf die Rohseite 4 des Ringfilters 3. Die Spülflüssigkeit 60 bzw. die Trocknungsluft 61 durchströmt dann den Ringfilter 3 und gelangt auf die Reinseite 5 des Ringfilters 3.

Das Gehäuse 2 weist einen ersten Auslass 7 auf, an dem eine Adapterschienenleitung 8 angeschlossen ist, die als Vorrichtung zum Durchspülen bzw. Reinigen von Innenlumina medizinischer und/oder zahnmedizinischer Instrumente dient. Durch diese Adapterschienenleitung 8 kann Spülflüssigkeit 60 oder Trocknungsluft 61 zu einer Adapterschiene 9 strömen. Die Adapterschiene 9 weist zahlreiche Adapter 90 auf, an die einzelne medizinische und/oder zahnmedizinische Instrumente angeschlossen werden können. Der besseren Übersichtlichkeit halber sind nur einige wenige dieser Adapter 90 mit dem entsprechenden Bezugszeichen versehen. Spülflüssigkeit 60 oder Trocknungsluft 61, die durch die Adapter 90 zu diesen Instrumenten strömen kann, kann anschließend die Innenlumina der entsprechenden Instrumente durchströmen.

Das Gehäuse 2 weist ferner einen zweiten Auslass 10 auf, der mit der Rohseite 4 des Ringfilters 3 in Strömungsverbindung steht. Stromabwärts des zweiten Auslasses 10 ist ein Ventil 11 angeordnet. Wenn das Ventil 11 geschlossen ist, kann die Spülflüssigkeit 60 oder die Trocknungsluft 61 nur durch den ersten Auslass 7 aus dem Gehäuse 2 austreten. Ist das Ventil 11 hingegen geöffnet, kann die Spülflüssigkeit 60 oder die Trocknungsluft 61 zusätzlich durch den zweiten Auslass 10 und durch das Ventil 11 hindurch zu einem Spülarm 12 strömen. Dieser Spülarm dient dabei als Vorrichtung zum Reinigen von Außenseiten medizinischer und/oder zahnmedizinischer Instrumente.

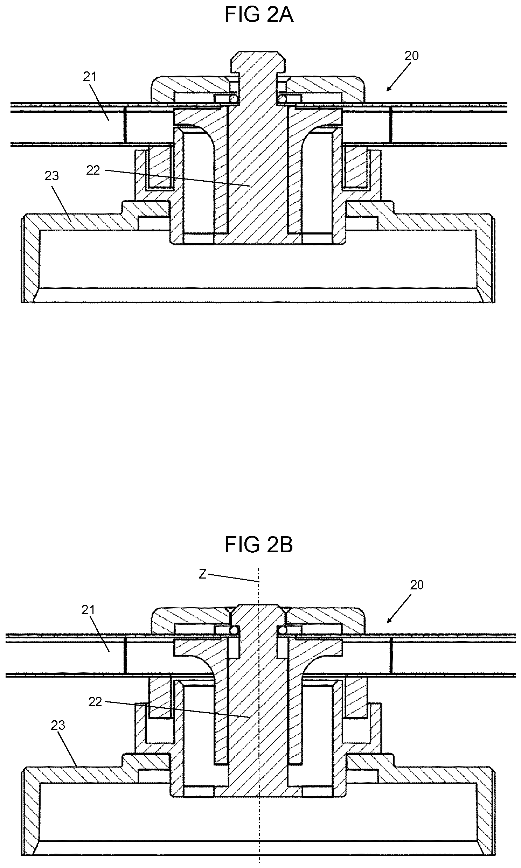

Das Ventil 11 ist als Spülarm-Drehführung in einem Deckel 13 des Gehäuses 2 ausgestaltet. Ein Ausführungsbeispiel für eine entsprechende Ventilausgestaltung ist in den Figuren 2A und 2B näher erläutert.

So zeigt die Figur 2 A eine Spülarmanordnung 20 mit einem Spülarm 21 und einer Drehführung 22, die als Spülarmaufnahme dient. Die Drehführung 22 ist Teil eines Deckels 23, der auf einem in der Figur 2A nicht dargestellten Gehäuse einer Filtereinrichtung dient (vergleiche diesbezüglich die Erläuterungen zur Figur 1). Wenn kein Spülflüssigkeitsdruck auf die Spülarmanordnung 20 einwirkt, sorgt das Eigengewicht des Spülarms 21 dafür, dass ein aus dem Spülarm 21 und der Drehführung 22 gebildetes Ventil geschlossen ist. Wenn in diesem geschlossenen Zustand des Ventils Spülflüssigkeit in die Filtereinrichtung einströmt, muss diese Spülflüssigkeit die Filtereinrichtung durch den Filter und den ersten Auslass wieder verlassen (vergleiche hierzu auch die Erläuterungen zur Figur 1). Dann erfolgt ausschließlich eine Innenreinigung von Instrumenten, die in einem Reinigungs- und Desinfektionsgerät aufgenommen sind, in dem die entsprechende Spülarmanordnung 20 ausgebildet ist.

Wenn sich der Spülflüssigkeitsdruck erhöht, öffnet das Ventil selbsttätig. Dies ist in der Figur 2B dargestellt. So wird der Spülarm 21 entgegen der Schwerkraft durch eine Axialverschiebung entlang einer Rotationsachse Z angehoben (dies entspricht in der Darstellung der Figur 2B einer Bewegung nach oben). Dadurch wird innerhalb der Drehführung 22 eine Strömungsverbindung zwischen einem Einlass der Drehführung 22 und einem Kanal, der die Drehführung 22 mit einem Innenraum des Spülarms 21 verbindet, hergestellt. Folglich kann Spülflüssigkeit durch die Drehführung 22 hindurch in das Innere des Spülarms 21 eintreten und aus dem Spülarm 21 durch entsprechende Spülöffnungen austreten.

Wenn der Flüssigkeitsdruck wieder abnimmt, fällt der Spülarm 21 durch die Schwerkraft automatisch wieder in seine geschlossene Position zurück.

Die in den Figuren 2A und 2B gezeigte Spülarmanordnung 20 ist eine besonders geeignete Realisierungsvariante für ein Ventil, das in einer Vorrichtung zum Reinigen von Außenseiten medizinischer und/oder zahnmedizinischer Instrumente verwirklicht ist.

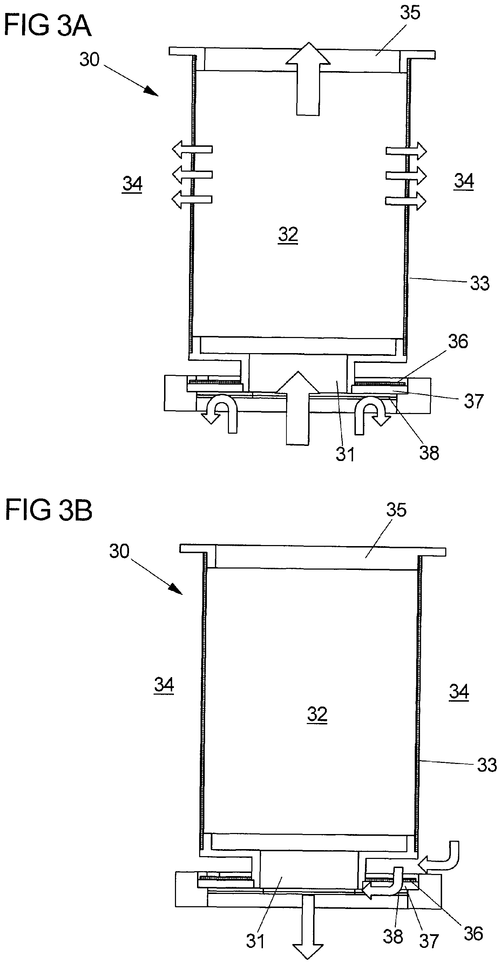

Die Figur 3A zeigt ein weiteres Ausführungsbeispiel einer Filtereinrichtung 30, wobei mittels Pfeilen die Strömung von Spülflüssigkeit oder Trocknungsluft durch diese Filtereinrichtung 30 visualisiert ist. Zunächst tritt Spülflüssigkeit oder Trocknungsluft durch einen Einlass 31 in den Innenraum 32 der Filtereinrichtung 30 ein. Der Innenraum 32 stellt zugleich eine Rohseite eines Filterelements 33 dar, das im Ausführungsbeispiel der Figur 3A wiederum als Ringfilterelement ausgestaltet ist. Die Spülflüssigkeit oder Trocknungsluft kann den Innenraum 32 nach Durchströmen des Ringfilters 33 über einen in der Figur 3A nicht dargestellten ersten Auslass wieder verlassen. Dieser Strömungspfad ist durch die sechs kleinen Pfeile in horizontaler Richtung dargestellt. Dabei kommt es zu einer Filtration der Spülflüssigkeit bzw. der Trocknungsluft, da der erste Auslass mit einer Reinseite 34 des Filterelements 33 in Strömungsverbindung steht.

Es ist aber auch möglich, dass die Spülflüssigkeit oder Trocknungsluft den Innenraum 32 durch einen zweiten Auslass 35 verlässt. Dies ist allerdings nur dann möglich, wenn ein in der Figur 3A nicht dargestelltes Ventil den zweiten Auslass 35 nicht verschließt, sondern ein Ausströmen von Spülflüssigkeit oder Trocknungsluft durch den zweiten Auslass 35 gestattet. Wenn die Spülflüssigkeit oder Trocknungsluft die Filtereinrichtung 30 durch den zweiten Auslass 35 verlässt, wird sie nicht filtriert. Vielmehr kann sie auf diesem Strömungspfad, der durch die dicken vertikalen Pfeile dargestellt ist, Schmutzpartikel, die sich auf einer Innenseite des Filterelements 33 abgelagert haben, abtragen. Diese Schmutzpartikel werden dann zu einer Vorrichtung zum Reinigen von Außenseiten medizinischer und/oder zahnmedizinischer Instrumente befördert. Dabei kann es sich beispielsweise um einen Spülarm handeln, wie er in den Figuren 1, 2A und 2B näher dargestellt ist.

Die Filtereinrichtung 30 weist ferner ein zusätzliches Filterelement 36 auf, das in einem zusätzlichen Auslass 37 angeordnet ist. Zum Verschließen dieses zusätzlichen Auslass 37 ist darüber hinaus eine Rückschlagmembran 38 vorgesehen. Wenn die Spülflüssigkeit oder Trocknungsluft wie in der Figur 3A durch die Filtereinrichtung 30 strömt, wird die Rückschlagmembran 38 gegen den zusätzlichen Auslass 37 gedrückt, sodass keine Spülflüssigkeit oder Trocknungsluft durch den zusätzlichen Auslass 37 strömen kann. Der zusätzliche Filter 36 stellt insoweit nur ein zusätzliches Sicherungselement dar, falls die Rückschlagmembran 38 nicht ordnungsgemäß funktionieren sollte.

In der Figur 3B werden die Strömungsverhältnisse von Spülflüssigkeit durch die Filtereinrichtung 30 für den Fall dargestellt, in dem die Umwälzpumpe, mit der die Spülflüssigkeit durch die Filtereinrichtung 30 befördert wird, gestoppt wurde. Dies ist immer dann der Fall, wenn keine weitere Spülflüssigkeit mehr gefördert werden soll, beispielsweise am Ende einer Reinigungsphase eines Reinigungsverfahrens. Wenn keine Spülflüssigkeit mehr gefördert wird, kommt es zu einem (geringen) Rückfluss von Spülflüssigkeit durch das Filterelement 33.

Um jedoch für ein schnelles und effektives Abfließen von überschüssiger Spülflüssigkeit zu sorgen, kann diese durch den zusätzlichen Auslass 37 und den zusätzlichen Filter 36 wieder aus der Filtereinrichtung 30 ausströmen. Denn wenn die Spülflüssigkeit in dieser "umgekehrten" Richtung fließt, drückt sie die Rückschlagmembran 38 nach unten, sodass der zusätzliche Auslass 37 geöffnet wird. Da die derart rückfließende Spülflüssigkeit bereits zuvor filtriert wurde, kommt es zu keiner weiteren Partikelanlagerung am zusätzlichen Filter 36. Vielmehr fließt die rückströmende Spülflüssigkeit im Gegenfluss durch den zusätzlichen Filter 36 und kann etwaige Verunreinigungen, die sich auf der Rohseite des zusätzlichen Filters 36 abgelagert haben könnten, wieder entfernen. Diese Rohseite entspricht der in der Figur 3B dargestellten Unterseite des zusätzlichen Filters 36.

## Patentansprüche

1. Reinigungs- und Desinfektionsgerät für medizinische und/oder zahnmedizinische Instrumente, mit einer Filtereinrichtung (1, 30) zum Filtrieren von Spülflüssigkeit, wobei die Filtereinrichtung (1, 30) ein Filterelement (3, 33) mit einer Rohseite (4, 32) und einer Reinseite (5, 34), einen Einlass (6, 31) zum wahlweisen Zuführen von Spülflüssigkeit (60) oder Trocknungsluft (61) in die Filtereinrichtung (1, 30), einen ersten Auslass (7) sowie einen zweiten Auslass (10, 35) aufweist, wobei der Einlass (6, 31) und der zweite Auslass (10, 35) mit der Rohseite (4, 32) des Filterelements (3, 33) in Strömungsverbindung stehen und wobei der erste Auslass (7) mit der Reinseite (5, 34) des Filterelements (3, 33) in Strömungsverbindung steht, wobei der erste Auslass (7) mit einer Vorrichtung (9) zum Durchspülen von Innenlumina medizinischer und/oder zahnmedizinischer Instrumente in Strömungsverbindung steht, wobei der zweite Auslass (10, 35) mit einer Vorrichtung (12, 21) zum Reinigen von Außenseiten medizinischer und/oder zahnmedizinischer Instrumente in Strömungsverbindung bringbar ist und wobei in einem durch den zweiten Auslass (10, 35) führenden Strömungspfad eine Ventileinrichtung (11) angeordnet ist, die ein Strömen von Spülflüssigkeit durch den zweiten Auslass (10, 35) hindurch verhindern oder gestatten kann.

2. Reinigungs- und Desinfektionsgerät nach Anspruch 1, **dadurch gekennzeichnet, dass** die Vorrichtung (12, 21) zum Reinigen von Außenseiten medizinischer und/oder zahnmedizinischer Instrumente derart ausgestaltet ist, dass sie eine Strömung von Spülflüssigkeit durch sie hindurch gestatten oder verhindert verhindern kann, und somit zumindest einen Teil der Ventileinrichtung (11) bildet.

3. Reinigungs- und Desinfektionsgerät nach Anspruch 1 oder 2, **dadurch gekennzeichnet, dass** die Vorrichtung (12, 21) zum Reinigen von Außenseiten medizinischer und/oder zahnmedizinischer Instrumente ein Spülarm ist.

4. Reinigungs- und Desinfektionsgerät nach Anspruch 3, **dadurch gekennzeichnet, dass** die Filtereinrichtung (3, 33) einen Deckel (13, 23) mit einer integrierten Spülarmaufnahme (22) zum beweglichen Befestigen des Spülarms an der Filtereinrichtung (1, 30) aufweist.

5. Reinigungs- und Desinfektionsgerät nach einem der vorhergehenden Ansprüche, **dadurch gekennzeichnet, dass** das Filterelement (3, 33) als Ringfilterelement ausgestaltet ist, wobei eine Innenseite des Ringfilterelements die Rohseite (4, 32) und eine Außenseite des Ringfilterelements die Reinseite (5, 34) des Ringfilterelements ist.

6. Reinigungs- und Desinfektionsgerät nach Anspruch 5, **dadurch gekennzeichnet, dass** der Einlass (6, 31) auf einer ersten Stirnseite des Ringfilterelements und der zweite Auslass (10, 35) auf einer der ersten Stirnseite gegenüberliegenden zweiten Stirnseite des Ringfilterelements angeordnet sind.

7. Reinigungs- und Desinfektionsgerät nach einem der vorhergehenden Ansprüche, **dadurch gekennzeichnet, dass** die Ventileinrichtung (11) einen Öffnungsdruck aufweist, der größer als ein Strömungswiderstand des Filterelements (3, 33) und des ersten Auslasses (7) ist.

8. Reinigungs- und Desinfektionsgerät nach einem der vorhergehenden Ansprüche, **dadurch gekennzeichnet, dass** die Filtereinrichtung (1, 30) einen zusätzlichen Auslass (37) in einem Bodenbereich der Filtereinrichtung (1, 30) aufweist, durch die rücklaufende Spülflüssigkeit aus der Filtereinrichtung (1,30) ausströmen kann.

9. Reinigungs- und Desinfektionsgerät nach Anspruch 8, **dadurch gekennzeichnet, dass** im zusätzlichen Auslass (37) ein zusätzliches Filterelement (36) angeordnet ist, das von Spülflüssigkeit, die durch den zusätzlichen Auslass (37) strömt, durchströmt werden muss.

10. Reinigungs- und Desinfektionsgerät nach Anspruch 8 oder 9, **dadurch gekennzeichnet, dass** der zusätzliche Auslass (37) mit einem Rückschlagelement (38) ausgestattet ist, das ein Durchströmen des zusätzlichen Auslasses (37) mit Spülflüssigkeit, die durch den Einlass in die Filtereinrichtung strömen soll, verhindert.

11. Verfahren zum Betrieb eines Reinigungs- und Desinfektionsgeräts für medizinische und/oder zahnmedizinische Instrumente nach einem der vorhergehenden Ansprüche, wobei das Reinigungs- und Desinfektionsgerät eine Filtereinrichtung (1, 30) zum Filtrieren von Spülflüssigkeit aufweist, wobei die Filtereinrichtung (1, 30) ein Filterelement (3, 33) mit einer Rohseite (4, 32) und einer Reinseite (5, 34), einen Einlass (6, 31) zum wahlweisen Zuführen von Spülflüssigkeit (60) oder Trocknungsluft (61) in die Filtereinrichtung (1, 30), einen ersten Auslass (7) sowie einen zweiten Auslass (10, 35) aufweist, wobei der Einlass (6, 31) und der zweite Auslass (10, 35) mit der Rohseite (4, 32) des Filterelements (3, 33) in Strömungsverbindung stehen und wobei der erste Auslass (7) mit der Reinseite (5, 34) des Filterelements (3, 33) in Strömungsverbindung steht, wobei Spülflüssigkeit (60), die durch den Einlass (6, 31) in die Filtereinrichtung (1, 30) einströmt, durch das Filterelement (3, 33) hindurch und durch den ersten Auslass (7) aus der Filtereinrichtung (1, 30) heraus- und zu einer Vorrichtung (9) zum Durchspülen von Innenlumina medizinischer und/oder zahnmedizinischer Instrumente hinströmt, wobei eine Ventileinrichtung (11) in einem geschlossenen Zustand den zweiten Auslass (10, 35) verschließt, wobei die Ventileinrichtung (11) in Abhängigkeit von Verfahrensparametern geöffnet wird, so dass die Spülflüssigkeit (60) dann zusätzlich durch den zweiten Auslass (10, 35) heraus- und zu einer Vorrichtung (12, 21) zum Reinigen von Außenseiten medizinischer und/oder zahnmedizinischer Instrumente hinströmt.

12. Verfahren nach Anspruch 11, **dadurch gekennzeichnet, dass** die Ventileinrichtung (11) in Abhängigkeit des Drucks der Spülflüssigkeit oder der Trocknungsluft, der Temperatur der Spülflüssigkeit oder der Trocknungsluft und/oder der Zeit elektromechanisch oder rein mechanisch geöffnet und/oder geschlossen wird.

13. Verfahren nach Anspruch 11 oder 12, **dadurch gekennzeichnet, dass** die Ventileinrichtung (11) dann, wenn ein Flüssigkeitsdruck der Spülflüssigkeit einen Schwellenwert überschreitet, geöffnet wird.

14. Verfahren nach Anspruch 13, **dadurch gekennzeichnet, dass** sich die Ventileinrichtung automatisch schließt, wenn der Flüssigkeitsdruck unter den Schwellenwert sinkt.

15. Verfahren nach Anspruch 13 oder 14, **dadurch gekennzeichnet, dass** Trocknungsluft (61) in die Filtereinrichtung (1, 30) mit einem Druck eingebracht wird, der unterhalb des Schwellenwerts liegt, so dass die Ventileinrichtung (11) geschlossen bleibt.

## Claims

1. Cleaning and disinfection device for medical and/or dental instruments, comprising a filter device (1, 30) for filtering rinsing liquid, wherein the filter device (1, 30) comprises a filter element (3, 33) having a raw side (4, 32) and a clean side (5, 34), an inlet (6, 31) for selectively supplying rinsing liquid (60) or drying air (61) into the filter device (1, 30), a first outlet (7), and a second outlet (10, 35), wherein the inlet (6, 31) and the second outlet (10, 35) are in flow communication with the raw side (4, 32) of the filter element (3, 33) and wherein the first outlet (7) is in flow communication with the clean side (5, 34) of the filter element (3, 33), wherein the first outlet (7) is is in flow communication with a device (9) for flushing out inner lumens of medical and/or dental instruments, wherein the second outlet (10, 35) can be brought into flow communication with a device (12, 21) for cleaning outer sides of medical and/or dental instruments and wherein a valve device (11) is arranged in a flow path leading through the second outlet (10, 35), which valve device can prevent or allow a flow of rinsing liquid through the second outlet (10, 35).

2. Cleaning and disinfection device according to claim 1, **characterized in that** the device (12, 21) for cleaning outer sides of medical and/or dental instruments is configured such that it can allow or prevent a flow of rinsing liquid therethrough, and thus forms at least a part of the valve device (11).

3. Cleaning and disinfection device according to claim 1 or 2, **characterized in that** the device (12, 21) for cleaning outer sides of medical and/or dental instruments is a rinsing arm.

4. Cleaning and disinfection device according to claim 3, **characterized in that** the filter device (3, 33) comprises a cover (13, 23) having an integrated rinsing arm receptacle (22) for movably fastening the rinsing arm to the filter device (1, 30).

5. Cleaning and disinfection device according to any of the preceding claims, **characterized in that** the filter element (3, 33) is configured as an annular filter element, wherein an inner side of the annular filter element is the raw side (4, 32) and an outer side of the annular filter element is the clean side (5, 34) of the annular filter element.

6. Cleaning and disinfection device according to claim 5, **characterized in that** the inlet (6, 31) is arranged on a first end face of the annular filter element, and the second outlet (10, 35) is arranged on a second end face of the annular filter element that is opposite the first end face.

7. Cleaning and disinfection device according to any of the preceding claims, **characterized in that** the valve device (11) has an opening pressure which is greater than a flow resistance of the filter element (3, 33) and of the first outlet (7).

8. Cleaning and disinfection device according to any of the preceding claims, **characterized in that** the filter device (1, 30) comprises an additional outlet (37) in a base region of the filter device (1, 30), through which outlet (37) the returning rinsing liquid can flow out of the filter device (1, 30).

9. Cleaning and disinfection device according to claim 8, **characterized in that** an additional filter element (36) is arranged in the additional outlet (37), through which filter element (36) the rinsing liquid flowing through the additional outlet (37) has to flow.

10. Cleaning and disinfection device according to claim 8 or 9, **characterized in that** the additional outlet (37) is equipped with a non-return element (38) which prevents a flow through the additional outlet (37) with rinsing liquid, which is intended to flow through the inlet into the filter device.

11. Method for operating a cleaning and disinfection device for medical and/or dental instruments according to any one of the preceding claims, wherein the cleaning and disinfection device comprises a filter device (1, 30) for filtering rinsing liquid, wherein the filter device (1, 30) comprises a filter element (3, 33) having a raw side (4, 32) and a clean side (5, 34), an inlet (6, 31) for selectively supplying rinsing liquid (60) or drying air (61) into the filter device (1, 30), a first outlet (7), and a second outlet (10, 35), wherein the inlet (6, 31) and the second outlet (10, 35) are in flow communication with the raw side (4, 32) of the filter element (3, 33) and wherein the first outlet (7) is in flow communication with the clean side (5, 34) of the filter element (3, 33), wherein rinsing liquid (60) flowing through the inlet (6, 31) into the filter device (1, 30) flows through the filter element (3, 33) and through the first outlet (7) out of the filter device (1, 30) and to a device (9) for flushing out inner lumens of medical and/or dental instruments, wherein a valve device (11) closes the second outlet (10, 35) in a closed state, wherein the valve device (11) is opened depending on method parameters, such that the rinsing liquid (60) then additionally flows out through the second outlet (10, 35) and to a device (12, 21) for cleaning outer sides of medical and/or dental instruments.

12. Method according to claim 11, **characterized in that** the valve device (11) is opened and/or closed electromechanically or purely mechanically, depending on the pressure of the rinsing liquid or of the drying air, the temperature of the rinsing liquid or of the drying air, and/or the time.

13. Method according to claim 11 or 12, **characterized in that** the valve device (11) is opened when a liquid pressure of the rinsing liquid exceeds a threshold value.

14. Method according to claim 13, **characterized in that** the valve device closes automatically when the liquid pressure drops below the threshold value.

15. Method according to claim 13 or 14, **characterized in that** drying air (61) is introduced into the filter device (1, 30) at a pressure which is below the threshold value, such that the valve device (11) remains closed.

## Revendications

1. Appareil de nettoyage et de désinfection pour des instruments médicaux et/ou dentaires, avec un dispositif filtrant (1, 30) pour filtrer du liquide de rinçage, dans lequel le dispositif filtrant (1, 30) présente un élément filtrant (3, 33) avec un côté brut (4, 32) et un côté propre (5, 34), une entrée (6, 31) pour amener au choix du liquide de rinçage (60) ou de l'air de séchage (61) dans le dispositif filtrant (1, 30), une première sortie (7) et une deuxième sortie (10, 35), dans lequel l'entrée (6, 31) et la deuxième sortie (10, 35) se trouvent en communication d'écoulement avec le côté brut (4, 32) de l'élément filtrant (3, 33) et dans lequel la première sortie (7) se trouve en communication d'écoulement avec le côté propre (5, 34) de l'élément filtrant (3, 33), dans lequel la première sortie (7) se trouve en communication d'écoulement avec un dispositif (9) pour le rinçage de lumières internes d'instruments médicaux et/ou dentaires, dans lequel la deuxième sortie (10, 35) peut être amenée en communication d'écoulement avec un dispositif (12, 21) pour nettoyer des côtés extérieurs d'instruments médicaux et/ou dentaires et dans lequel un dispositif de soupape (11) est disposé dans un trajet d'écoulement menant à travers la deuxième sortie (10, 35), , dans lequel le dispositif de soupape (11) peut empêcher ou permettre un écoulement de liquide de rinçage à travers la deuxième sortie (10, 35) de part en part.

2. Appareil de nettoyage et de désinfection selon la revendication 1, **caractérisé en ce que** le dispositif (12, 21) pour nettoyer des côtés extérieurs d'instruments médicaux et/ou dentaires est configuré de telle manière qu'il peut permettre ou empêcher un écoulement de liquide de rinçage à travers lui de part en part et forme ainsi au moins une partie du dispositif de soupape (11).

3. Appareil de nettoyage et de désinfection selon la revendication 1 ou 2, **caractérisé en ce que** le dispositif (12, 21) pour nettoyer des côtés extérieurs d'instruments médicaux et/ou dentaires est un bras de rinçage.

4. Appareil de nettoyage et de désinfection selon la revendication 3, **caractérisé en ce que** le dispositif filtrant (3, 33) présente un couvercle (13, 23) avec un logement de bras de rinçage (22) intégré pour fixer de manière mobile le bras de rinçage sur le dispositif filtrant (1, 30).

5. Appareil de nettoyage et de désinfection selon l'une quelconque des revendications précédentes, **caractérisé en ce que** l'élément filtrant (3, 33) est configuré en tant qu'élément filtrant annulaire, dans lequel un côté intérieur de l'élément filtrant annulaire est le côté brut (4, 32) et un côté extérieur de l'élément filtrant annulaire est le côté propre (5, 34) de l'élément filtrant annulaire.

6. Appareil de nettoyage et de désinfection selon la revendication 5, **caractérisé en ce que** l'entrée (6, 31) est disposée sur un premier côté frontal de l'élément filtrant annulaire et la deuxième sortie (10, 35) est disposée sur un deuxième côté frontal, faisant face au premier côté frontal, de l'élément filtrant annulaire.

7. Appareil de nettoyage et de désinfection selon l'une quelconque des revendications précédentes, **caractérisé en ce que** le dispositif de soupape (11) présente une pression d'ouverture, qui est supérieure à une résistance d'écoulement de l'élément filtrant (3, 33) et de la première sortie (7).

8. Appareil de nettoyage et de désinfection selon l'une quelconque des revendications précédentes, **caractérisé en ce que** le dispositif filtrant (1, 30) présente une sortie (37) supplémentaire dans une zone de fond du dispositif filtrant (1, 30), par lequel du reflux de liquide de rinçage peut s'écouler hors du dispositif filtrant (1, 30).

9. Appareil de nettoyage et de désinfection selon la revendication 8, **caractérisé en ce que** dans la sortie (37) supplémentaire est disposé un élément filtrant (36) supplémentaire, qui doit être traversé par du liquide de rinçage, qui s'écoule à travers la sortie (37) supplémentaire.

10. Appareil de nettoyage et de désinfection selon la revendication 8 ou 9, **caractérisé en ce que** la sortie (37) supplémentaire est équipée d'un élément anti-retour (38), qui empêche un écoulement du liquide de rinçage, qui doit s'écouler dans le dispositif filtrant par l'entrée, à travers la sortie (37) supplémentaire.

11. Procédé pour faire fonctionner un appareil de nettoyage et de désinfection pour des instruments médicaux et/ou dentaires selon l'une quelconque des revendications précédentes, dans lequel l'appareil de nettoyage et de désinfection présente un dispositif filtrant (1, 30) pour filtrer du liquide de rinçage, dans lequel le dispositif filtrant (1, 30) présente un élément filtrant (3, 33) avec un côté brut (4, 32) et un côté propre (5, 34), une entrée (6, 31) destinée à amener au choix du liquide de rinçage (60) ou de l'air de séchage (61) dans le dispositif filtrant (1, 30), une première sortie (7) et une deuxième sortie (10, 35), dans lequel l'entrée (6, 31) et la deuxième sortie (10, 35) se trouvent en communication d'écoulement avec le côté brut (4, 32) de l'élément filtrant (3, 33) et dans lequel la première sortie (7) se trouve en communication d'écoulement avec le côté propre (5, 34) de l'élément filtrant (3, 33), dans lequel du liquide de rinçage (60), qui afflue dans le dispositif filtrant (1, 30) par l'entrée (6, 31), sort du dispositif filtrant (1, 30) par l'élément filtrant (3, 33) de part en part et par la première sortie (7) et s'écoule en direction d'un dispositif (9) destiné à rincer des lumières intérieures d'instruments médicaux et/ou dentaires, dans lequel un dispositif de soupape (11) ferme la deuxième sortie (10, 35) dans un état fermé, dans lequel le dispositif de soupape (11) est ouvert en fonction de paramètres de procédé de sorte que le liquide de rinçage (60) sort alors en plus par la deuxième sortie (10, 35) et s'écoule en direction d'un dispositif (12, 21) destiné à nettoyer des côtés extérieurs d'instruments médicaux et/ou dentaires.

12. Procédé selon la revendication 11, **caractérisé en ce que** le dispositif de soupape (11) est ouvert et/ou est fermé de manière électromécanique ou de manière purement mécanique en fonction de la pression du liquide de rinçage ou de l'air de séchage, de la température du liquide de rinçage ou de l'air de séchage et/ou du temps.

13. Procédé selon la revendication 11 ou 12, **caractérisé en ce que** le dispositif de soupape (11) est ouvert lorsqu'une pression de liquide du liquide de rinçage dépasse une valeur de seuil.

14. Procédé selon la revendication 13, **caractérisé en ce que** le dispositif de soupape se ferme automatiquement lorsque la pression de liquide chute sous la valeur de seuil.

15. Procédé selon la revendication 13 ou 14, **caractérisé en ce que** de l'air de séchage (61) est introduit dans le dispositif filtrant (1, 30) avec une pression, qui est inférieure à la valeur de seuil, de sorte que le dispositif de soupape (11) reste fermé.
